# EUROPEAN PATENT SPECIFICATION

(11) **EP 1 006 890 B1**
(45) Date of publication and mention of the grant of the patent: **20.09.2006**
(21) Application number: 98937238.8
(22) Date of filing: 29.07.1998
(51) Int. Cl.: A61B 17/12

(54) **OCCLUSION SYSTEM FOR ANEURYSM REPAIR**
OKKLUSSIONSSYSTEM ZUM AUSBESSERN EINES ANEURYSMAS
SYSTEME D'OCCLUSION SERVANT A REPARER UN ANEVRISME

(30) Priority: 04.08.1997 US 54810 P
(43) Date of publication of application: 14.06.2000
(73) Proprietor: Boston Scientific Limited, Barbados, West Indies (BB)
(72) Inventor: McCRORY, Jennifer, J., Sunnyvale, CA 94086 (US); GUTERMAN, Lee, R., Amherst, NY 14226 (US); TREMAGLIO, Anthony R. Jr., Brookline, MA 02146 (US)
(74) Representative: Cross, Rupert Edward Blount
(86) International application number: PCT/US1998/015690
(87) International publication number: WO 1999/005977

(56) References cited:
- EP-A- 0 743 047
- WO-A-94/06460
- WO-A-97/26939
- WO-A-97/31672
- FR-A- 2 641 692

## Description

The present invention deals with a system for treating an aneurysm. More specifically, the present invention is directed to an occlusion system for deployment of an occlusion device within the aneurysm.

An aneurysm is a localized stretching or distension of an artery due to a weakening of the vessel wall. For example, congenital "berry" aneurysms, i.e., small spherical distensions, occur in the vessels of the brain. The distensions -- often referred to as the aneurysm sac -- are related to defects in the muscular coating of the artery and are probably developmental in origin. Rupture of aneurysms account for the majority of spontaneous hemorrhages. Approximately 25,000 intracranial aneurysms rupture every year in North America.

Several methods of treating aneurysms have been attempted, with varying degrees of success. At present, the treatment of aneurysms with drugs is substantially ineffective. Also, extra-vascular surgery, referred to as open craniotomy, for the purpose of preserving the parent artery is replete with disadvantages. A patient subject to open craniotomy for intercranial aneurysms typically must undergo general anesthesia, surgical removal of part of the skull, brain retraction, dissection around the neck of the sac, and placement of a clip on the parent artery to prevent rebleeding.

Alternative treatments include endovascular occlusion where the interior of the aneurysm is entered with a guidewire or a microcatheter. An occlusion is formed within the sac with an intention to preserve the parent artery. A preferred means for forming a mass is through the introduction of an embolic agent within the sac. Examples of embolic agents include a detachable coil, which is detached from the end of a guidewire, and a liquid polymer which polymerizes rapidly on contact with blood to form a firm mass. The embolic agent may initially generate a thrombotic mass in the sac as well. The thrombotic mass is composed of the elements of blood, namely platelets, fibrin, red cells and leukocytes. However, the thrombotic mass may typically dissipate through the normal lysing process.

Endovascular occlusion is not without drawbacks. For example, there is a risk of overfilling the sac and consequent embolic agent migration into the parent vessel. This results in occlusion of the parent artery and could lead to distal embolization. Further, there is a risk of the embolic agent becoming dislodged from hemodynamic forces, which can result in distal embolization, restricted blood flow in the parent artery or total occlusion of the parent artery.

Moreover, endovascular occlusion can be ineffective when the neck of an aneurysm is not well defined because the risk of embolic agent migration is greater with such a sac. Prior art methods used to reduce the risk of embolic agent migration in an ill-defined neck include blockage of the parent artery with a device inside the vasculature to isolate the sac from circulation while the occlusion is formed. However, blockage of the parent artery can itself be highly undesirable, but necessary in certain circumstances when the aneurysm presents a greater risk to the patient. After the occlusion is formed in the cavity, and circulation is restored in the parent artery, an ill-defined neck increases the risk of the embolic agent becoming dislodged.

International patent application no. WO-A-97/26939 describes an aneurysm occlusion device and represents prior art within the meaning of Rule 29 (1)(a)EPC.

### SUMMARY OF THE INVENTION

A first aspect of the present invention provides a device for treating an aneurysm in a parent vessel according to claim 1. The device preferably includes a collapsible member which is permeable to blood flow.

A second aspect of the present invention provides a system for treating an aneurysm in a vessel according to claim 12.

### BRIEF DESCRIPTION OF THE DRAWINGS

FIG. 1 is a side view of an occlusion device deployed within an aneurysm.
FIGS. 2A - 2D show the deployment of the occlusion device of FIG. 1 within the aneurysm.
FIGS. 3A - 3C show the deployment of an embolic agent in cooperation with the device of FIG. 1 within the aneurysm.
FIG. 4 is a side view of another occlusion device deployed within an aneurysm.
FIGS. 5A - 5C show the deployment of the occlusion device of FIG. 4.
FIG. 6 shows the occlusion device of FIG. 4 in cooperation with an embolic agent within the aneurysm.
FIG. 7 is a side view of another occlusion device deployed within the aneurysm.
FIG. 8 shows the deployment of the device of FIG. 7.
FIG. 9 is a side view of another occlusion device deployed within an aneurysm.
FIG. 10 is a top view of the device of FIG. 9.
FIGS. 11A-11D illustrate the deployment of the device in FIG. 9.

### DETAILED DESCRIPTION OF THE PREFERRED EMBODIMENTS

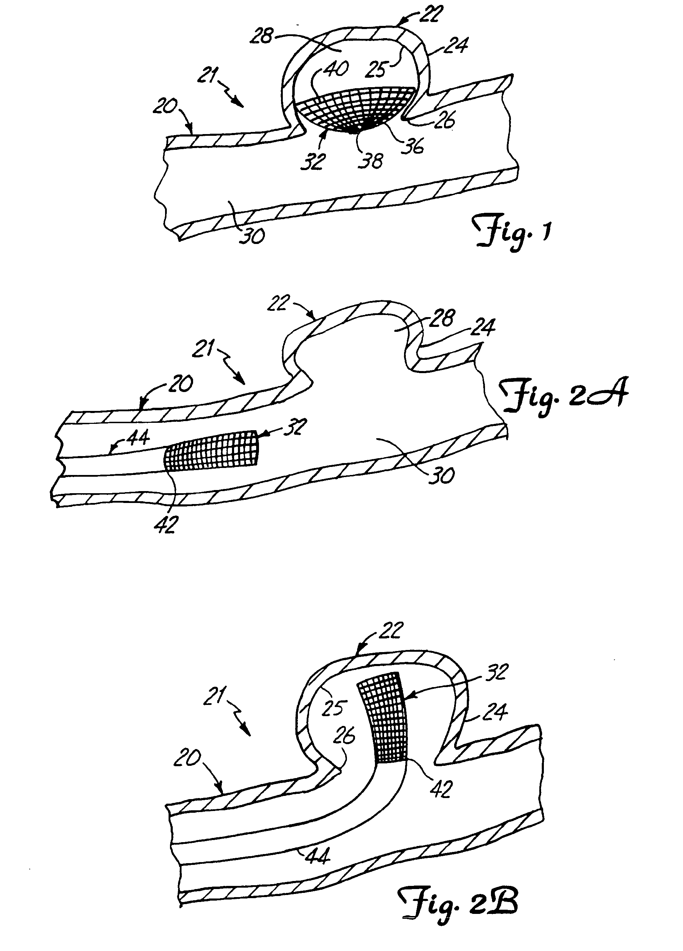

FIG. 1 shows parent vessel 20 sectioned for clarity within a vascular region 21 of the body. The vessel 20 has an aneurysm 22 with a sac 24, inner wall 25 and neck 26. The sac 24 forms a vascular cavity 28 in communication with the lumen 30 of vessel 20. Occlusion device 32, embodying the present invention, is deployed in the vascular cavity 28 proximate the neck 26.

The device 32 is preferably a flexible structure capable of bridging the neck 26 from within the aneurysm 22. In the illustrated embodiment, device 32 does not interfere with the lumen 30 of parent vessel 20. The device 32 is formed in the shape of a dish, and includes a plurality of struts 36 which project radially from the centre 38. The device 32 also includes a plurality of parabolic rings 40 which lend stability to the structure. The struts 36 may be formed of any suitable material, but in the preferred embodiment are formed from metal and covered with fabric. Rather than rings of fabric, the struts may be covered with a mesh, not shown. Alternatively, the device 32 may be formed of a polymeric or similar material.

Device 32 can be deployed in the aneurysm 22 in a variety of ways. FIGS. 2A - 2D show the deployment of device 32 in accordance with the present invention. FIG. 2A shows device 32 collapsed and attached to the distal end 42 of delivery catheter 44 inside lumen 30. Device 32 is a shape memory structure which is capable of residing in a collapsed state but then expands to its dish-like state in response to an appropriate stimulus. The device 32 can be made of thermo-sensitive material which is flexible below a transition temperature and readily collapsible, but which is less resilient or generally rigid above the transition temperature. In the preferred embodiment, the struts 36 may be formed of small diameter nitinol wire which has the shape memory properties described above.

As indicated in FIG. 2A, the device 32 is collapsed and delivered through the vasculature 21 in a more flexible state, below its transition temperature. FIG. 2B shows the collapsed device 32 within the aneurysm 22 and positioned there with the delivery catheter 44. The delivery catheter 44 can be used to manipulate and adjust the position of device 32 within the aneurysm 22 before and/or after it is expanded.

Once in place within the aneurysm 22, the temperature of the vascular region is raised from a point below the transition temperature to a point above the transition temperature. This can be accomplished, for instance, by injecting warm saline or the like into the vascular region, or simply by letting the occlusion device 32 warm to body temperature. Also, device 32 can be delivered while being flushed with a cold saline solution to maintain the temperature of device 32 below its transition temperature. Once device 32 is in place, the cold saline flush is discontinued and device 32 warms to a temperature above the transition temperature. After the occlusion device 32 reaches the transition temperature, it expands to the predetermined diameter or width and makes contact with the inner walls 25 and the neck 26.

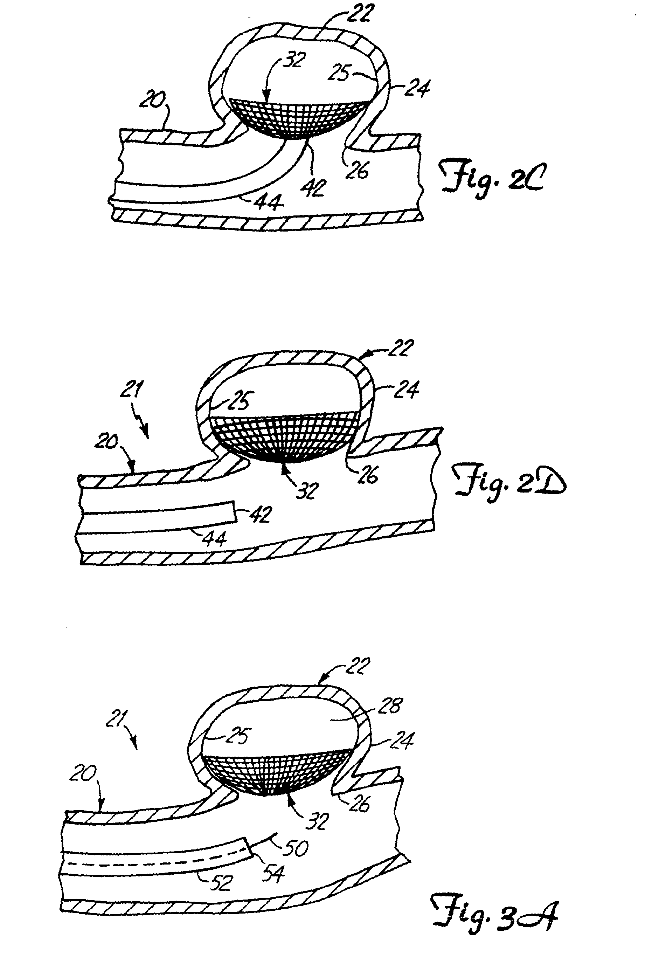

FIG. 2C shows the device 32 expanded to bridge neck 26 of the aneurysm 22. The delivery catheter 44 is then detached from the device 32 which remains in place within the aneurysm 22. Detachment can be accomplished in any number of suitable ways. For instance, device 32 can be attached with a soluble adhesive and detached by injecting a suitable solvent. Also, device 32 can be attached by a frictional fit and detached by exerting a force on catheter 44. Further, device 32 can be detached electrolytically in the same fashion as a Guglielmi detachable coil (GDC). FIG. 2D shows device 32 permanently in position within the aneurysm 22 with the delivery catheter 44 removed.

Device 32 can also be formed of a resilient material which is permanently biased in the deployed position. In order to collapse device 32 for introduction into the vasculature, a filament or other fibrous thread can be removably wound about the exterior of device 32 maintaining it in a collapsed position. Once device 32 is introduced into cavity 28, the thread is removed, allowing device 32 to deploy outwardly. Removal of the thread is preferably accomplished by exerting gentle traction on the thread which causes it to disengage from device 32.

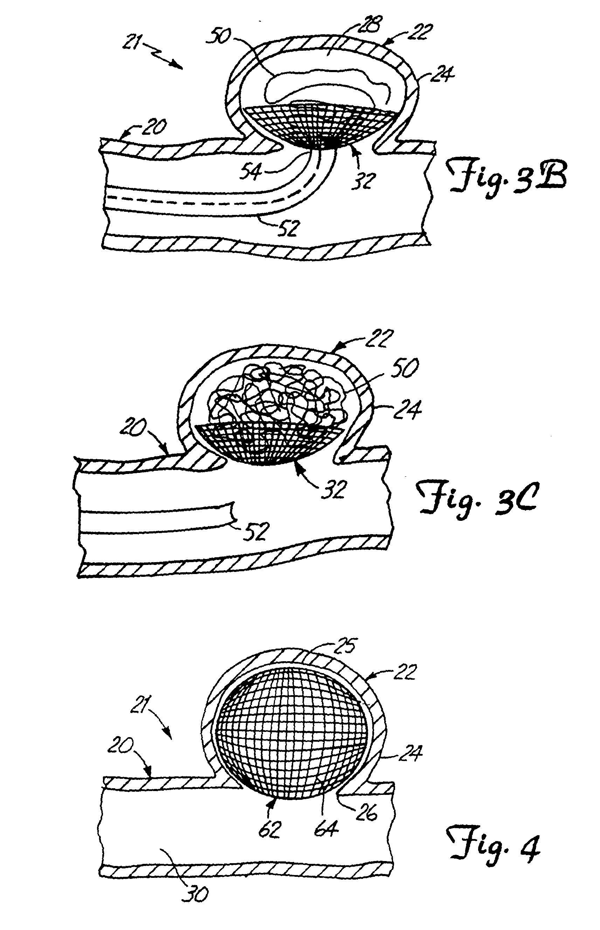

FIGS. 3A-3C show the delivery of an embolic agent 50 through the device to form a thrombotic mass within the aneurysm 22. FIG. 3A shows a microcatheter 52 having the embolic agent 50 at the distal tip 54 of the catheter 52. The embolic agent 50, such as a stainless steel coil or a liquid polymer, or a combination of solid and liquid embolic agents (e.g., a coil and a liquid polymer) is delivered to the aneurysm via the microcatheter. FIG. 3B shows the injection of the embolic agent 50 within the aneurysm 22. Distal tip 54 is positioned against the device 32 such that the embolic agent 50 extends into the cavity 28. Distal tip 54 and device 32 preferably include markings thereon such that they can be viewed during the procedure using conventional fluoroscopy, x-ray or ultrasound, or other suitable techniques, or device 32 and distal tip 54 are viewed with the aid of a suitable scope or fiber optic bundle.

The permeable nature of the device 32 allows the distal tip of delivery catheter 52 to be positioned behind device 32 in cavity 28. The delivery catheter 52 is movable relative to device 32 to allow precise placement of the distal tip of the catheter within the cavity 28. Catheter tip placement influences the quality of filling, and the adjustable nature improves the ability to fill the aneurysm 22. As the embolic agent 50 is injected into the cavity 28, the blood within the aneurysm 22 escapes through the neck 26 and through the holes in device 32. As the embolic agent 50 fills cavity 28, it acts to further secure the device 32 in place in the neck 26. It is to be understood that the embolic agent 50 may be deployed either prior to detachment of the delivery catheter 44 or after detachment.

FIG. 3C shows the embolic agent 50 packed within the aneurysm 22, held in place with the occlusion device 32, and with the delivery catheter 52 removed. The device 32 and embolic agent 50 cooperate to repair the aneurysm. The embolic agent 50 secures the device 32 within the sac 24 in the region of neck 26 and prevents the device 32 from being pushed further into the sac 24 by hemodynamic forces. The device 32 traps the embolic agent 50, permits a tighter packing of the embolic agent 50, and reduces the likelihood of migration of the embolic agent 50 out of aneurysm 22 into parent vessel 20. This helps prevent distal embolization.

Also, the presence of the device 32 at the neck 26 provides a scaffolding for tissue to grow and create a new endoluminal surface inside the vessel 20 to isolate the aneurysm 22 from the circulation of blood within the vasculature 21. The device 32 may be combined with biologic materials such as collagen, fibrin, or the like, to facilitate both thrombosis and cell infiltration and fibrotic tissue growth over the neck 26 to create the new parent artery endoluminal surface.

It should be noted that device 32 is preferably formed of a continuous structure as shown in FIGS. 1-3C. However, it can also be formed of several leaflet members connected by a hinge connection at a center portion of device 32 and configured to fold out upon deployment within aneurysm 22.

FIG. 4 shows an illustration of another occlusion device 62 in accordance with the present invention (generally referred to as a spherical occlusion device) where like portions of the vasculature 21 are referred to by like reference numerals. Spherical occlusion device 62 is similar to device 32 shown in FIG. 1 in that it is to be deployed within the aneurysm 22 and is generally permeable to blood flow. However, the spherical device 62 includes a collapsible spherical mesh structure 64 that not only covers the neck 26 of aneurysm 22 but provides stability to the entire inner wall 25 of the aneurysm 22. The structure 64 can be fashioned from the same materials used to make device 32, as described above.

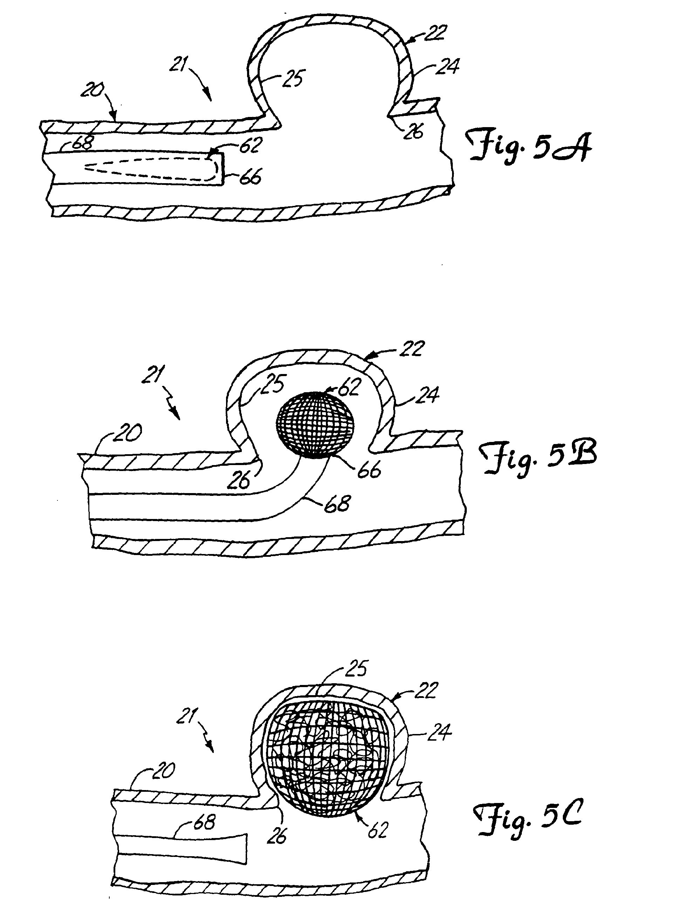

FIGS. 5A-5C show the deployment of the spherical device 62 in accordance with the present invention. FIG. 5A shows the collapsed device 62 inserted within the distal tip 66 of a delivery catheter 68 rather than attached thereto. FIG. 5B shows the placement of the distal tip 66 within the aneurysm 22. The device 62 is then advanced out of catheter 68 and into the aneurysm 22 and allowed to expand and bridge the neck 26 of the aneurysm 22. FIG. 5C shows the expanded spherical device 62 within the aneurysm 22 with the catheter 68 removed. It is to be understood that this deployment method is suitable for other occlusion devices in accordance with the present invention and not limited to the spherical device.

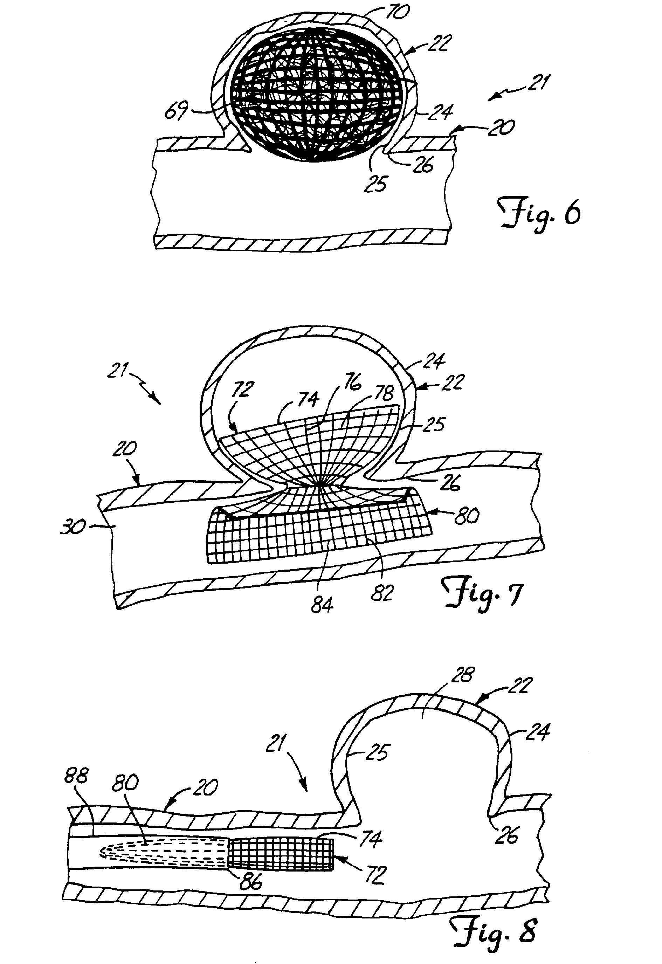

After the device 62 is expanded within the aneurysm 22, a microcatheter can be used in the fashion described above to deliver an embolic agent within the spherical device. Alternatively, the same catheter used to deliver spherical device 62 can be used to deliver the embolic agent. FIG. 6 shows the embolic agent 69 packed within the spherical device 62. The spherical device 62 provides support for the entire aneurysm wall 25 during the delivery of the embolic agent 69 and thereafter. This allows tighter packing of the embolic agent 69 with a reduced likelihood of overflow, and reduced likelihood of rupture particularly at the typically fragile dome region 70 of the aneurysm 22.

FIG. 7 shows another occlusion device 72 constructed in accordance with the present invention. The device 72 includes a distal portion 74 which can be similar to the dish-like device 32 shown in FIG. 1 or in the shape of a sphere (not shown). The distal portion 74 preferably includes a nitinol wire frame 76 and covered with a mesh 78, or the like. The distal portion 74 is deployed within the aneurysm 22 and is preferably secured against the inner wall 25 of the aneurysm 22 at the neck 26. A proximal portion 80 includes a frame 82 attached to the frame 76 of the distal portion 74. The frame 82 can be covered with a mesh 84. Preferably, the proximal portion 80 is expandable to conform to the inner wall of the parent vessel 20. In one preferred embodiment, the distal portion 74 is coated or treated with thrombogenic agents in a manner known to those skilled in the art to facilitate thrombosis within the aneurysm 22. The proximal portion 80, on the other hand, can be coated with non-thrombogenic agents which reduce the likelihood of thrombosis within the parent vessel 20. Once seated in the neck 26 of aneurysm 22, the distal portion 74 inhibits the migration of the device 72 into the parent vessel 20. The proximal portion 80 inhibits the migration of the device 72 into the aneurysm sac 24.

FIG. 8 shows the preferred means of deployment of the device 72. The proximal portion 80 is collapsed and inserted into the distal tip 86 of a delivery catheter 88. The distal portion 74 may also be inserted within the delivery catheter 88 or can extend past the distal tip 86. During deployment, the distal portion 74 is positioned with the catheter 88 to be within the aneurysm 22, and is then permitted to expand (by advancing it out of the end of catheter 88, or by application of some other suitable stimulus). Distal portion 74 is preferably configured so that it can be deployed from within catheter 88, retracted back into catheter 88 and redeployed. The proximal portion 80 is then advanced out of, and detached from, the delivery catheter 88. Again, this detachment can be accomplished by any suitable system such as electrolytic detachment from a delivery member or catheter in the same fashion as a GDC coil. Delivery catheter 88 is then removed from the vascular region 21 to allow the proximal portion 80 to expand and conform to the shape of the inner wall 25 of the parent vessel 20.

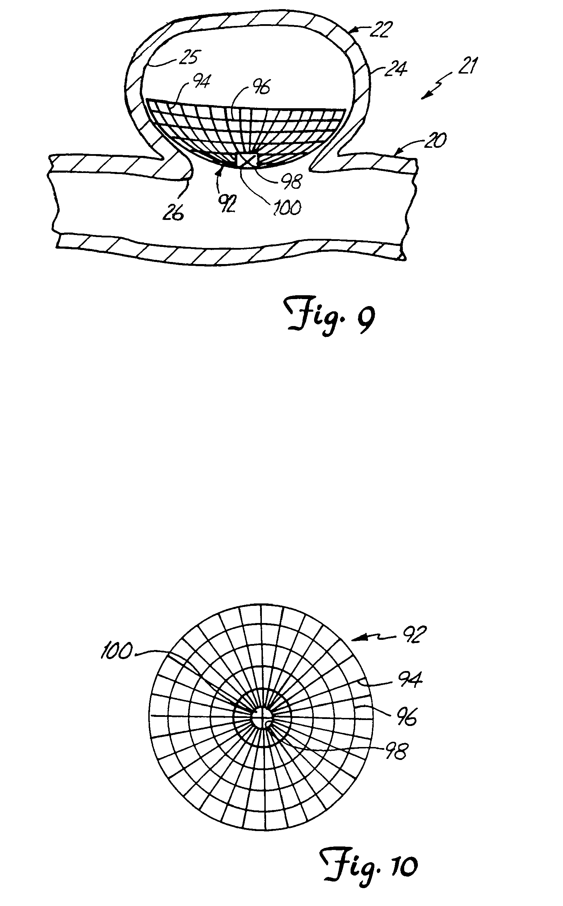

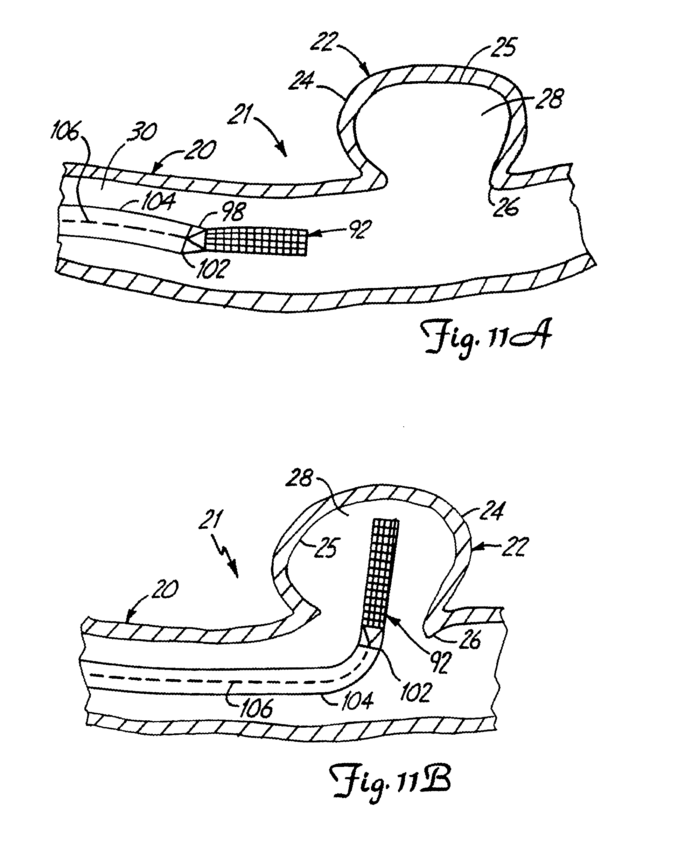

FIG. 9 shows a view of another occlusion device 92 constructed in accordance with the present invention and deployed within the aneurysm 22. Device 92 is formed in the shape of a parabolic dish and is characterized by a plurality of struts 94, or frame, and is covered by a mesh 96 similar to that of the device 32 shown in FIG. 1. However, the device 92 includes a small valve 98, such as a mitervalve, attached to the frame 94 at the center 100 of the dish.

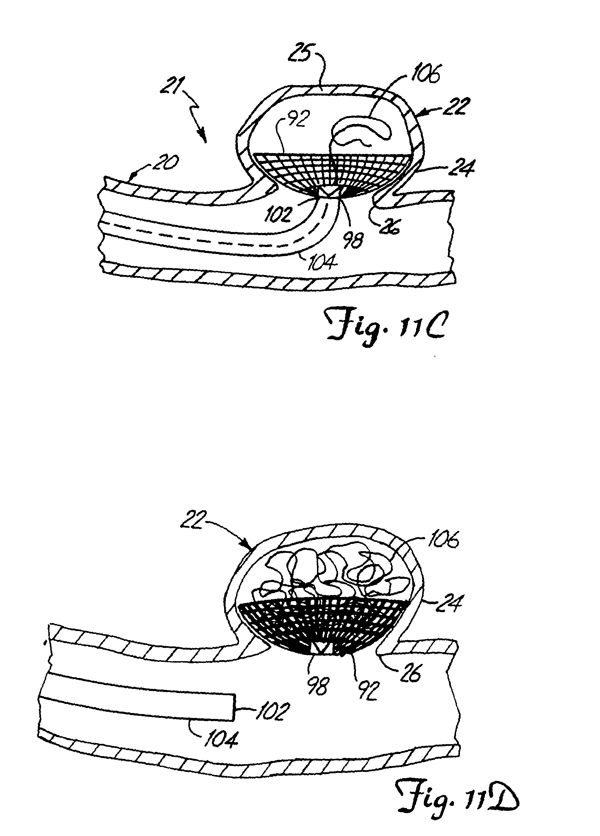

FIG. 10 shows a top view of the device 92 of FIG. 9. Nitinol struts 94 extend from the mitervalve 98 to form a generally circular shape. Preferably, the mesh 96 does not cover the mitervalve 98.

FIGS. 11A-11D show the deployment of the device 92 in accordance with the present invention. FIG. 11A shows the collapsed device 92 attached to the distal tip 102 of a catheter 104. The distal tip 102 of the catheter 104 is inserted through the mitervalve 98. An embolic agent 106 is contained within the catheter 104 for injection therethrough. FIG. 11B shows the collapsed device 92 positioned within the aneurysm 22. Once within the aneurysm 22, the device 92 is allowed to expand. FIG. 11C shows the expanded device 92 in position within the aneurysm 22. Once the device 92 is expanded to bridge the neck 26 of the aneurysm 22, the embolic agent 106 is injected into the aneurysm 22 through the mitervalve 98. FIG. 11D shows the device 92 and embolic agent 106 within the aneurysm 22 with the catheter 104 removed. After the embolic agent 106 is deployed, gentle traction of the catheter 104 disengages the distal tip 102 from the mitervalve 98 which allows removal of catheter 104. As with the other embodiments, other suitable detachment techniques can be used including electrolytic detachment.

It should be noted that the devices described herein can be coated with a number of suitable coatings. Among the coatings which could be applied are growth factors. A number of suitable growth factors include vascular endothelial growth factor (VEGF), platelet derived growth factor (PDGF), vascular permeability growth factor (VPF), basic fibroblast growth factor (bFGF), and transforming growth factor beta (TGF-beta).

## Claims

1. A device (32) for treating an aneurysm (22) in a parent vessel (20), the parent vessel defining a lumen (30), the aneurysm having a neck (26) and an inner wall (25) defining a cavity (28) communicating with the lumen (30), the device (32) configured for deployment within the cavity (28) and comprising:
a member (32) having a collapsed state when the device is delivered and arranged such that when the device is deployed the member (32) expands to bridge the neck (26) of the aneurysm (22) and is in contact with the inner wall (25);
**characterised in that** the member (32) comprises either:
(a) an expandable wire frame covered by a permeable mesh; or
(b) an expandable frame having a covering attached thereto and a valve (98) attached to the frame (92);
to allow an embolic agent (50) to be injected through the member by a microcatheter after the device is deployed to form a thrombotic mass within the aneurysm (22).

2. The device of claim 1 wherein the member (32) is permeable to blood flow.

3. The device of claim 1 wherein the expandable frame includes a plurality of generally parabolic wire struts (36) configured in the shape of a dish (32).

4. The device of claim 1 wherein the expandable frame includes a plurality of struts (64) configured in the shape of a sphere (62).

5. The device of claim 1 wherein the member further comprises a proximal portion (80) having a proximal expandable frame (82) attached to the expandable frame (76), the proximal expandable frame (82) configured for deployment within the lumen.

6. The device of claim 5 wherein the distal expandable frame (76) includes a plurality of parabolic struts configured in the shape of a dish.

7. The device of claim 6 wherein the proximal expandable frame (82) is expandable to contact the parent vessel (20).

8. The device of claim 7 wherein the proximal expandable frame (82) is coated with a non-thromobogenic agent.

9. The device of claim 1 wherein the expandable wire frame is made from a shape-memory material.

10. The device of claim 1 wherein member (32) is coated with a thrombogenic agent.

11. The device of claim 1 wherein the member (32) has a covering layer thereon formed of a vascular growth factor.

12. A system for treating an aneurysm (22) in a vessel (20), the aneurysm (22) having an inner wall (25) and a neck (26) defining a cavity (28), the system comprising:
a collapsible member (32) attached to a delivery catheter (44);
wherein the collapsible member (32) is configured to be endovascularly delivered to the aneurysm (22) for deployment therein;
wherein the collapsible member (32) is expandable within the cavity (28) so as to bridge the neck (26) and contact the inner wall (25);
**characterised in that** the system includes a microcatheter (52) configured to have an embolic agent (50), to be positioned against said collapsible member (32), and to inject said embolic agent (50) through said collapsible member (32) into the cavity (28) to form a mass, and **in that** the collapsible member (32) comprises either:
(a) an expandable wire frame covered by a permeable mesh; or
(b) an expandable frame having a covering attached thereto and a valve (98) attached to the frame (92);
to allow the embolic agent (50) to be injected through the member (32) by the microcatheter after the device is deployed.

## Patentansprüche

1. Vorrichtung (32) zur Behandlung eines Aneurysmas (22) in einem Stammgefäß (20), wobei das Stammgefäß ein Lumen (30) definiert, das Aneurysma einen Hals (26) und eine Innenwand (25) aufweist, die eine mit dem Lumen (30) in Verbindung stehende Höhle (28) definieren und die Vorrichtung (32) zur Entwicklung innerhalb der Höhle (28) ausgebildet ist, umfassend:
ein Element (32), das in einem zusammengefalteten Zustand vorliegt, wenn die Vorrichtung eingebracht und derart angeordnet ist, dass sich bei der Anwendung der Vorrichtung das Element (32) aufweitet, so dass der Hals (26) des Aneurysmas (22) überbrückt wird und die Innenwand (25) berührt,
**dadurch gekennzeichnet, dass** das Element (32) entweder umfasst:
(a) ein aufweitbares drahtartiges Gerüst, das mit einer durchlässigen Netzstruktur bedeckt ist, oder
(b) ein aufweitbares Gerüst mit einer daran angebrachten Abdeckung und einem an dem Gerüst (92) angebrachten Ventil (98),
so dass ein embolisches Agens (50) durch das Element über einen Mikrokatheter nach dem Anwenden der Vorrichtung injiziert werden kann, um eine thrombotische Masse innerhalb des Aneurysmas (22) zu bilden.

2. Vorrichtung nach Anspruch 1, wobei das Element (32) für den Blutstrom durchlässig ist.

3. Vorrichtung nach Anspruch 1, wobei das aufweitbare Gerüst eine Vielzahl von im Wesentlichen parabolförmigen drahtartigen Verstrebungen (36) umfasst, welche die Form einer Schale (32) bilden.

4. Vorrichtung nach Anspruch 1, wobei das aufweitbare Gerüst eine Vielzahl von Verstrebungen (64) umfasst, welche die Form einer Kugel (62) bilden.

5. Vorrichtung nach Anspruch 1, wobei das Element weiter einen proximalen Teil (80) mit einem proximalen aufweitbaren Gerüst (82) umfasst, das an dem aufweitbaren Gerüst (76) angebracht ist und das proximale aufweitbare Gerüst (82) zur Entwicklung innerhalb des Lumens ausgebildet ist.

6. Vorrichtung nach Anspruch 5, wobei das distale aufweitbare Gerüst (76) eine Vielzahl von parabolförmigen Verstrebungen umfasst, welche die Form einer Schale bilden.

7. Vorrichtung nach Anspruch 6, wobei das proximale aufweitbare Gerüst (82) aufweitbar ist, um in Kontakt mit dem Stammgefäß (20) gebracht zu werden.

8. Vorrichtung nach Anspruch 7, wobei das proximale aufweitbare Gerüst (82) mit einem nicht-thrombogenen Agens beschichtet ist.

9. Vorrichtung nach Anspruch 1, wobei das aufweitbare drahtartige Gerüst aus einem Material mit Formgedächtnis hergestellt ist.

10. Vorrichtung nach Anspruch 1, wobei das Element (32) mit einem thrombogenen Agens beschichtet ist.

11. Vorrichtung nach Anspruch 1, wobei das Element (32) eine aus einem vaskulären Wachstumsfaktor gebildete Abdeckschicht besitzt.

12. System zur Behandlung eines Aneurysmas (22) in einem Gefäß (20), wobei das Aneurysma (22) eine Innenwand (25) und einen Hals (26) aufweist, die eine Höhle (28) bilden, wobei das System umfasst:
ein zusammenfaltbares Element (32), das mit einem Abgabekatheter (44) verbunden ist,
wobei das zusammenfaltbare Element (32) derart ausgebildet ist, dass es an das Aneurysma (22) zur darin vorgesehenen Entwicklung endovaskulär abgegeben wird,
wobei das zusammenfaltbare Element (32) innerhalb der Höhle (28) aufweitbar ist, so dass es den Hals (26) überbrückt und die Innenwand (25) berührt,
**dadurch gekennzeichnet, dass** das System einen Mikrokatheter (52) mit einem embolischen Agens (50) umfasst, das gegen das zusammenfaltbare Element (32) gerichtet ist und das embolische Agens (50) über das zusammenfaltbare Element (32) in die Höhle (28) zur Bildung einer Masse injiziert wird und dass das zusammenfaltbare Element (32) entweder umfasst:
(a) ein aufweitbares drahtartiges Gerüst, das mit einer permeablen Netzstruktur bedeckt ist, oder
(b) ein aufweitbares Gerüst mit einer daran angebrachten Abdeckung und einem an dem Gerüst (92) angebrachten Ventil (98),
so dass das embolische Agens (50) durch das Element über den Mikrokatheter nach dem Anwenden der Vorrichtung injiziert werden kann.

## Revendications

1. Dispositif (32) pour traiter un anévrisme (22) dans un vaisseau porteur (20), le vaisseau porteur définissant une lumière (30), l'anévrisme comportant un collet (26) et une paroi interne (25) définissant une cavité (28) communiquant avec la lumière (30), le dispositif (32) étant configuré pour un déploiement à l'intérieur de la cavité (28) et comprenant:
un élément (32) étant dans un état plié lorsque le dispositif est introduit et mis en place de telle sorte que lorsque le dispositif est déployé, l'élément (32) se déploie pour combler le collet (26) de l'anévrisme (22) et est en contact avec la paroi interne (25) ;
**caractérisé en ce que** l'élément (32) comprend soit:
(a) une structure constituée de fils pouvant être déployée recouverte par un treillis perméable; ou
(b) une structure pouvant être déployée possédant un revêtement sur celle-ci et une valve (98) attachée à la structure (92);
afin de permettre à un agent embolique (50) d'être injecté à travers l'élément par un microcathéter une fois que le dispositif est déployé pour former une masse thrombotique à l'intérieur de l'anévrisme (22).

2. Dispositif selon la revendication 1, dans lequel l'élément (32) est perméable à la circulation sanguine.

3. Dispositif selon la revendication 1, dans lequel la structure pouvant être déployée comprend une pluralité de montants de fils généralement paraboliques (36) configurés en forme d'assiette (32).

4. Dispositif selon la revendication 1, dans lequel la structure pouvant être déployée comprend une pluralité de montants (64) configurés en forme de sphère (62).

5. Dispositif selon la revendication 1, dans lequel l'élément comprend en outre une partie proximale (80) possédant une structure proximale pouvant être déployée (82) attachée à la structure pouvant être déployée (76), la structure proximale pouvant être déployée (82) étant configurée pour un déploiement à l'intérieur de la lumière.

6. Dispositif selon la revendication 5, dans lequel la structure distale pouvant être déployée (76) comprend une pluralité de montants paraboliques configurés en forme d'assiette.

7. Dispositif selon la revendication 6, dans lequel la structure proximale pouvant être déployée (82) peut être déployée pour être en contact avec le vaisseau porteur (20).

8. Dispositif selon la revendication 7, dans lequel la structure proximale pouvant être déployée (82) est recouverte par un agent non thrombogénique.

9. Dispositif selon la revendication 1, dans lequel la structure de fils pouvant être déployée est fabriquée avec un matériau ayant la mémoire des formes.

10. Dispositif selon la revendication 1, dans lequel l'élément (32) est recouvert par un agent thrombogène.

11. Dispositif selon la revendication 1, dans lequel l'élément (32) possède une couche de revêtement sur celui-ci qui est un facteur de croissance vasculaire.

12. Système pour traiter un anévrisme (22) dans un vaisseau (20), l'anévrisme (22) possédant une paroi interne (25) et un collet (26) définissant une cavité (28), le système comprenant:
un élément pliable (32) attaché à un cathéter d'introduction (44);
dans lequel l'élément pliable (32) est configuré pour être introduit par voie endovasculaire vers l'anévrisme (22) pour un déploiement dans celui-ci;
dans lequel l'élément pliable (32) peut être déployé à l'intérieur de la cavité (28) afin de combler le collet (26) et être en contact avec la paroi interne (25);
**caractérisé en ce que** le système comprend un microcathéter (52) configuré pour contenir un agent embolique (50), être positionné contre ledit élément pliable (32), et pour injecter ledit agent embolique (50) à travers ledit élément pliable (32) dans la cavité (28) pour former une masse, et **en ce que** l'élément pliable (32) comprend soit:
(a) une structure constituée de fils pouvant être déployée recouverte par un treillis perméable; ou
(b) une structure pouvant être déployée possédant un revêtement sur celle-ci et une valve (98) attachée à la structure (92) ;
pour permettre à un agent embolique (50) d'être injecté à travers l'élément (32) par un microcathéter une fois que le dispositif est déployé.
